Europäisches Patentamt

European Patent Office

Office européen des brevets

(19)

(11) Numéro de publication : **0 214 169 B1**

(12)

# FASCICULE DE BREVET EUROPEEN

(45) Date de publication du fascicule du brevet :
**24.07.91 Bulletin 91/30**

(51) Int. Cl.$^5$ : **A61B 5/11, A01K 29/00**

(21) Numéro de dépôt : **86901088.4**

(22) Date de dépôt : **07.02.86**

(86) Numéro de dépôt international :
**PCT/FR86/00034**

(87) Numéro de publication internationale :
**WO 86/04802 28.08.86 Gazette 86/19**

(54) **PROCEDE ET DISPOSITIF D'EVALUATION DES EFFETS PSYCHOTROPES DE SUBSTANCES MEDICAMENTEUSES CHEZ LES ANIMAUX.**

(30) Priorité : 26.02.85 FR 8502752

(43) Date de publication de la demande :
18.03.87 Bulletin 87/12

(45) Mention de la délivrance du brevet :
24.07.91 Bulletin 91/30

(84) Etats contractants désignés :
BE CH DE FR GB IT LI NL

(56) Documents cités :
EP-A- 0 087 015
DE-A- 1 598 465
DE-A- 2 256 859
FR-A- 1 519 720
GB-A- 1 061 338
GB-A- 2 058 359
US-A- 2 940 312

(73) Titulaire : STERU, Lucien
201, rue d'Alésia
F-75014 Paris (FR)
Titulaire : STERU, Marius
191, rue d'Alésia
F-75014 Paris (FR)

(72) Inventeur : STERU, Lucien
201, rue d'Alésia
F-75014 Paris (FR)
Inventeur : STERU, Marius
191, rue d'Alésia
F-75014 Paris (FR)

(74) Mandataire : Martin, Jean-Jacques et al
Cabinet REGIMBEAU 26, Avenue Kléber
F-75116 Paris (FR)

EP 0 214 169 B1

## Description

La présente invention concerne un procédé et un dispositif d'évaluation des effets psychotropes de substances médicamenteuses chez les animaux de laboratoire.

On entend par médicament psychotrope une substance susceptible de modifier favorablement un comportement pathologique. On considère comme effets psychotropes d'une substance les effets modifiant le comportement aussi bien dans le sens souhaitable (médicament) que dans un sens indésirable.

Une des applications importantes, mais non limitative, de la présente invention, est l'évaluation des effets anti-dépresseurs, ou au contraire dépresseurs, et des effets stimulants, ou au contraire sédatifs des molécules à action potentiellement pharmaceutique ou toxique.

Le document DE-A-2256859 décrit un dispositif pour la mesure de la variation de longueur d'un muscle isolé et de la force exercée par ce muscle, donc du travail qu'il fournit suite à une excitation électrique externe. Ce document propose plus précisément un dispositif adapté pour analyser soit des contractions isométriques, soit des contractions isotoniques. Ce dispositif comprend une enceinte apte à recevoir le muscle et contenant une solution nutritive thermostatée. Le dispositif comprend en outre deux pinces destinées à être fixées respectivement aux extrémités du muscle, deux électrodes pour l'excitation du muscle placées dans l'enceinte et deux systèmes de mesure associés respectivement aux pinces.

Un premier système de mesure est conçu pour mesurer la variation de longueur du muscle, sous charge constante. Ce premier système est formé d'un barreau rigide articulé à rotation, relié à une extrémité à une première des pinces et chargé à sa seconde extrémité par une masse constante et d'une bobine HF placé en regard du barreau pour détecter les déplacements de celui-ci. Des butées sont prévues pour limiter le déplacement du barreau. Le second système de mesure est conçu pour mesurer la force exercée par le muscle. Il comprend un barreau élastique associé à une bobine HF de détection. Le travail fourni est obtenu en multipliant le déplacement détecté par le premier système de mesure par la force détectée par le second système de mesure.

Diverses méthodes d'évaluation des effets psychotropes de substances médicamenteuses ont déjà été proposées. Ces méthodes cependant n'ont pas donné pleinement satisfaction.

Cela semble dû en partie au fait que les tests doivent d'une part être effectués sur un grand nombre d'animaux de laboratoire, tels que des rats ou des souris, et d'autre part être réalisés avec précision et reproductibilité pour permettre une exploitation des résultats de type statistique et rendre l'exploitation des résultats correctement prédictive des effets susceptibles d'être produits, par la substance testée, chez l'homme.

La présente invention vient améliorer la situation en proposant un nouveau procédé d'évaluation des effets psychotropes de substances médicamenteuses chez les animaux, qui élimine dans une large mesure les interventions d'un opérateur et améliore de ce fait les performances du test, tant sur le plan quantitatif en autorisant l'évaluation sur un nombre important d'animaux que sur le plan qualitatif en améliorant la sensibilité, la fiabilité et la reproductibilité. La présente invention permet en particulier de diminuer très notablement les perturbations introduites par l'environnement sur la mesure et notamment de réduire les perturbations induites par le manipulateur ainsi que les risques d'erreurs d'évaluation.

La présente invention permet enfin de réduire les coûts et les temps d'expérimentation. L'un des avantages essentiels de l'invention réside dans la possibilité d'automatiser quasi-totalement le processus d'évaluation.

Le procédé d'évaluation des effets psychotropes, conforme à la présente invention, est basé sur la constatation générale selon laquelle les mouvements d'un animal, tel qu'un rat ou une souris, auquel ont été administrées les substances médicamenteuses et qui a été ultérieurement suspendu, de préférence par la queue, ou le cas échéant par un membre ou une autre partie du corps, sont liés aux effets psychotropes desdites substances médicamenteuses.

En effet, les médicaments antidépresseurs ou stimulants, par exemple, diminuent les périodes d'immobilité ; les médicaments tranquillisants ou sédatifs au contraire augmentent les périodes d'immobilité.

Plus précisément, les inventeurs ont établi que pour réaliser un test représentatif il convenait de détecter les mouvements de l'animal essayant de se libérer qui sont liés à un déplacement du centre de gravité de l'animal dans la direction verticale c'est-à-dire qui sont liés à un déplacement qui implique une dépense importante d'énergie ; de tels mouvements seront dits "mouvements significatifs" dans la suite de la description. Dans la pratique, ces mouvements significatifs ne sont pas continus mais répartis en une pluralité de phases élémentaires d'agitation, d'amplitude et de durée variables. L'expression "agitation significative" sera utilisée dans la suite de la description pour désigner l'ensemble des mouvements significatifs de l'animal pendant un temps d'expérimentation déterminé.

Le procédé d'évaluation des effets psychotropes de substances médicamenteuses chez les animaux, conforme à la présente invention, comprend les étapes consistant, après avoir administré les substances médicamenteuses à un animal :

i) à suspendre celui-ci de préférence par la queue, sur un organe de suspension équipé d'un capteur sensible aux efforts verticaux transmis par l'animal à cet organe, lors de son agitation,

ii) à détecter, pendant un temps d'expérimentation prédéterminé de l'ordre des minutes ou de dizaines de minutes, le signal généré par le capteur, signal qui constitue la mesure des mouvements de l'animal liés à un déplacement du centre de gravité de celui-ci dans la direction verticale et représente par conséquent les mouvements significatifs de l'animal car impliquant une dépense importante d'énergie, et

iii) à déterminer, par traitement du signal généré par le capteur, la valeur d'un paramètre représentatif de l'agitation significative de l'animal pendant le temps d'expérimentation.

L'étape ii) de détection peut comprendre une simple mesure du signal issu du capteur, suivie d'un traitement direct pour la détermination de la valeur du paramètre représentatif de l'agitation significative, ou encore une mesure du signal suivie d'une mémorisation en vue d'un traitement ultérieur.

De préférence, le capteur est sensible à la flexion ou aux déplacements de l'organe de suspension dans la direction verticale.

Les inventeurs ont proposé différentes variantes pour déterminer la valeur du paramètre représentatif de l'agitation significative de l'animal pendant le temps d'expérimentation.

Selon une première variante, l'étape iii) de détermination de la valeur du paramètre représentatif de l'agitation significative de l'animal pendant le temps d'expérimentation consiste :

– à mesurer la durée des phases élémentaires de mouvement significatif de l'animal, détectées par le capteur et

– à réaliser la somme des durées des phases élémentaires de mouvement significatif détectées pendant le temps d'expérimentation, cette somme étant représentative de la valeur recherchée du paramètre.

Selon une seconde variante, l'étape iii) de détermination de la valeur du paramètre représentatif de l'agitation significative de l'animal pendant le temps d'expérimentation consiste :

a) à mesurer les durées des phases d'inactivité de l'animal, en considérant comme phase d'inactivité tout état de l'animal en dehors des phases de mouvement significatif, et

b) à réaliser la somme des durées les phases d'inactivité détectées pendant le temps d'expérimentation, cette somme étant considérée représentative de la valeur recherchée du paramètre.

Selon une troisième variante, l'étape iii) de détermination de la valeur du paramètre représentatif de l'agitation significative de l'animal pendant le temps d'expérimentation consiste :

a) à comparer le signal généré par le capteur à une valeur de seuil pour retenir les signaux représentatifs d'un mouvement significatif dont les amplitudes dépassent un seuil prédéterminé,

b) à mesurer les durées des phases de mouvements significatifs ou des phases d'inactivité à partir des signaux retenus à l'étape a) et

c) à réaliser la somme des durées des phases de mouvements significatifs ou des phases d'inactivité mesurées à l'étape b) pendant le temps d'expérimentation, ces sommes étant représentatives des valeurs recherchées du paramètre.

Cette variante permet d'éliminer les perturbations introduites par les mouvements internes cardio-respiratoires et autres de l'animal.

Selon une quatrième variante, l'étape iii) de détermination de la valeur du paramètre représentatif de l'agitation significative de l'animal pendant le temps d'expérimentation consiste :

a) à mesurer les amplitudes des signaux générés par le capteur, correspondant aux efforts verticaux exercés par l'animal sur l'organe de suspension pendant les mouvements élémentaires significatifs,

b) à élever les valeurs des amplitudes de ces signaux au carré ; ce carré est considéré ici comme représentatif de l'énergie dépensée par l'animal pendant chaque mouvement élémentaire significatif,

c) à opérer la somme des carrés obtenus à l'étape b) pour évaluer l'énergie totale dépensée pendant le temps d'expérimentation, cette énergie totale étant représentative de la valeur recherchée du paramètre.

Selon une cinquième variante, l'étape iii) de détermination de la valeur du paramètre représentatif de l'agitation significative de l'animal pendant le temps d'expérimentation consiste :

a) à mesurer les valeurs des amplitudes des signaux électriques générés par le capteur, correspondant aux efforts verticaux exercés sur l'organe de suspension par les mouvements significatifs de l'animal ;

b) à élever au carré les valeurs mesurées des amplitudes, ce carré étant considéré ici comme représentatif de l'énergie dépensée par l'animal pendant chaque mouvement significatif élémentaire ;

c) à effectuer la somme des carrés obtenus à l'étape b) afin d'évaluer l'énergie totale dépensée par l'animal pendant le temps d'expérimentation, et

d) à diviser la somme des carrés obtenue à l'étape c) par le temps total d'agitation significative de l'animal, la valeur de ce quotient étant considérée comme représentative de la valeur recherchée du paramètre.

Le temps total d'agitation significative de l'animal utilisé à l'étape d) ci-dessus peut être déterminé soit en effectuant la somme des durées des phases élémentaires de mouvements significatifs, soit en effectuant la différence entre le temps d'expérimentation prédéterminé et la somme des durées des phases d'inactivité détectées pendant le temps d'expérimentation.

Cette cinquième variante permet de mesurer la puissance (énergie par seconde) dépensée pendant les phases de mouvement significatif par l'animal.

Les cinq variantes précitées de détermination de la valeur du paramètre représentatif de l'agitation significative de l'animal peuvent par ailleurs être combinées.

La présente invention concerne également un dispositif d'évaluation des effets psychotropes de substances médicamenteuses chez les animaux pour la mise en oeuvre du procédé précité qui comprend :
   – au moins un dispositif de saisie d'informations comportant :
      . un organe de suspension muni de moyens aptes à permettre une suspension d'un animal, de préférence par la queue, et
      . un capteur sensible aux efforts verticaux transmis par l'animal à cet organe de suspension, lors de son agitation, et liés à un déplacement du centre de gravité de l'animal dans la direction verticale,
   – des moyens de traitement du signal généré par le capteur pendant un temps d'expérimentation prédéterminé, aptes à évaluer l'agitation significative de l'animal pendant ce temps, et
   – des moyens de visualisation d'un paramètre représentatif de la valeur de cette agitation significative.

Selon un premier mode de réalisation , l'organe de suspension comprend une barre flexible généralement horizontale fixée sur une embase par une première extrémité et munie de moyens de suspension de l'animal au voisinage de sa seconde extrémité, le capteur étant formé d'un transducteur sensible à la déformation de flexion verticale de la barre.

De préférence, le transducteur comprend au moins une jauge de contraintes.

Selon un second mode de réalisation, l'organe de suspension comprend une barre rigide généralement horizontale articulée sur une embase autour d'un axe horizontal transversal à sa direction longitudinale, par une première extrémité, et munie de moyens de suspension au voisinage de sa seconde extrémité, la barre étant associée à un élément élastique sollicitant la barre vers une position neutre de repos.

De façon avantageuse, selon l'invention, le dispositif comprend en outre un amortisseur limitant les oscillations mécaniques parasites.

Plus précisément encore, pour pouvoir expérimenter simultanément sur plusieurs animaux, pour faciliter l'exploitation statistique des résultats obtenus, le dispositif conforme à la présente invention pour l'évaluation des effets psychotropes de substances médicamenteuses chez les animaux comprend :

   – une pluralité d'organes de suspension munis chacun de moyens aptes à permettre la suspension d'un animal sur un organe respectif, de préférence par la queue,
   – des capteurs associés respectivement aux organes de suspension et sensibles aux efforts verticaux transmis par les animaux à ces organes de suspension, lors de leur agitation,
   – un organe de multiplexage recevant les signaux délivrés par les capteurs et qui attaque :
   – des moyens de traitement des signaux générés par ces capteurs pendant un temps d'expérimentation prédéterminé, aptes à évaluer l'agitation significative de chaque animal pendant ce temps, et
   – des moyens de visualisation des valeurs déterminés par les moyens de traitement.

Selon une autre caractéristique avantageuse de la présente invention, en particulier lors de tests sur des animaux relativement lourds, il est prévu des moyens servant de support à l'animal suspendu pour alléger l'effort dû au poids de l'animal exercé sur le membre suspendu.

D'autres caractéristiques et avantages de la présente invention apparaîtront à la lecture de la description détaillée qui va suivre et en regard des dessins annexés donnés à titre d'exemples non limitatifs, et sur lesquels :
   – la figure 1 représente une vue latérale schématique d'un premier mode de réalisation d'un dispositif de saisie d'informations conforme à la présente invention,
   – la figure 2 représente une vue latérale schématique d'un second mode de réalisation d'un dispositif de saisie d'informations conforme à la présente invention,
   – la figure 3 représente une vue en coupe verticale, selon un plan de symétrie, d'une variante du premier mode de réalisation précité, d'un dispositif de saisie d'informations, selon un plan de coupe référencé III-III sur la figure 4,
   – la figure 4 représente une vue latérale de cette même variante de réalisation selon une vue illustrée par la flèche référencée IV sur la figure 3,
   – la figure 5 représente une vue de dessus de cette même variante, selon une vue illustrée par la flèche référencée V sur la figure 3,
   – la figure 6 illustre schématiquement le circuit électronique de traitement des informations délivrées par un dispositif de saisie d'informations conforme à la présente invention comportant un nombre important de postes de test en vue d'une exploitation statistique,
   – la figure 7 représente la structure schématique d'un tel dispositif présentant plusieurs postes de tests, c'est-à-dire plusieurs postes aptes à recevoir, en suspension, un animal.

Les figures 8 et 9 représentent deux vues similaires aux figures 3 et 4 d'une variante de réalisation. comportant des moyens d'appui pour l'animal.

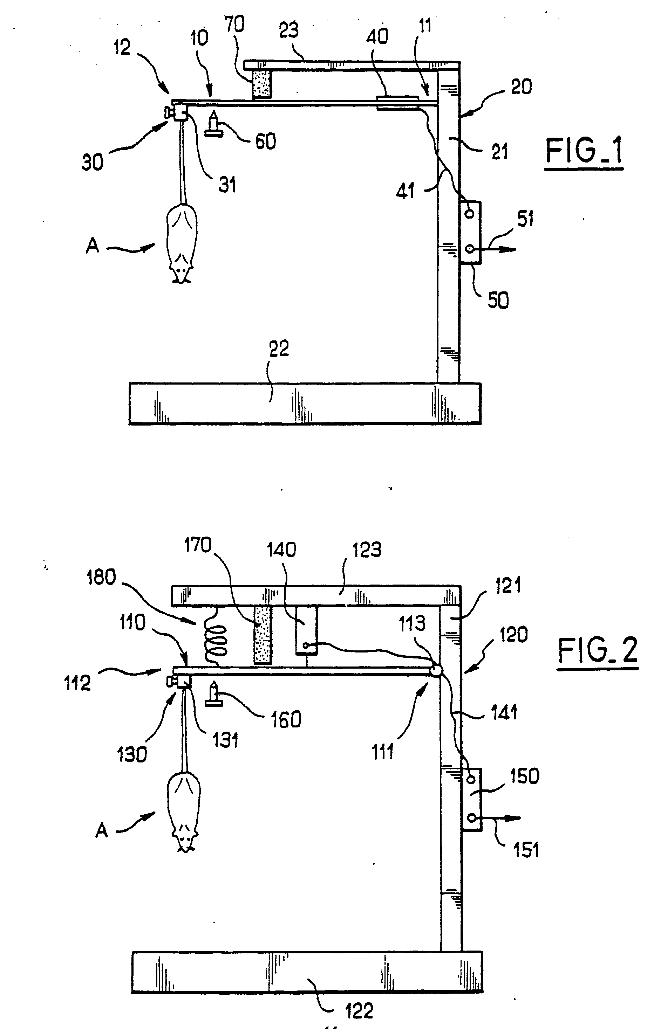

On va dans un premier temps décrire la structure du dispositif de saisie d'informations illustré sur la figure 1.

Celui-ci comprend une barre flexible 10 (formant organe de suspension), généralement horizontale, dont une première extrémité 11 est fixée, par encastrement, sur une console ou équivalent 20, et dont la seconde extrémité 12 est équipée d'un dispositif 30 pour la fixation (par suspension) de l'animal d'expérimentation A.

Selon le mode de réalisation représenté sur la figure 1, la barre 10 est encastrée dans un montant 21 généralement vertical supporté par une embase 22 horizontale.

Le dispositif 30 de fixation par suspension précité est susceptible d'être l'objet de nombreuses variantes de réalisation, classiques en elles-mêmes.

De préférence, ce dispositif de fixation 30 est adapté pour permettre l'accrochage de l'animal d'essai, de préférence souris ou rat, par la queue. Le cas échéant, le dispositif de fixation 30 peut être utilisé pour l'accrochage de l'animal d'essai par un membre ou par toute autre partie du corps.

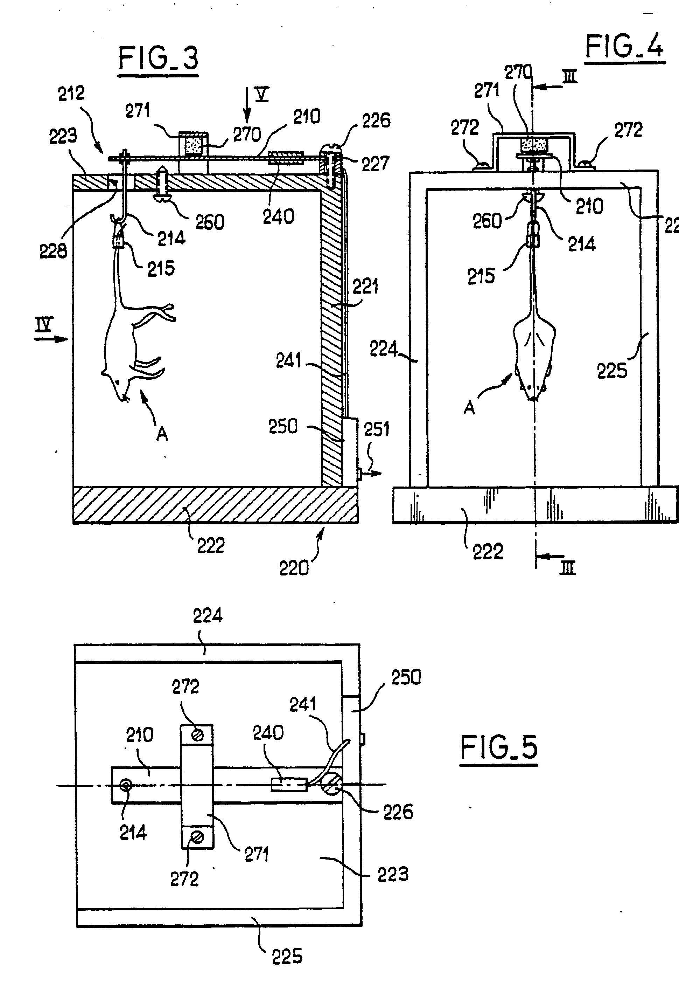

De façon schématique, ce dispositif peut comprendre un manchon à serrage 31 comme représenté sur la figure 1 ou une pince à ressort, ou encore un crochet 214(comme représenté sur la figure 3) sur lequel on accroche la queue ou un membre de l'animal par de la bande adhésive ou par une pince.

Un tel système pourrait être remplacé par autre moyen fonctionnellement équivalent.

De préférence, la section de la barre 10 est telle que les déformations de flexion dues à un effort vertical soient beaucoup plus importantes que celles dues à un effort horizontal. Pour cela, la barre 10 présente avantageusement une section rectangulaire dont le grand côté est orienté dans la direction horizontale (perpendiculairement au plan de la figure 1).

Comme représenté sur la figure 1, la barre 10 est en outre équipée d'au moins un transducteur 40 sensible aux efforts verticaux transmis par l'animal à cette barre, et plus précisément sensible à la déformation de flexion verticale de la barre 10.

Ce transducteur 40 peut comprendre au moins une jauge de contraintes, de préférence, il sera équipé de plusieurs jauges afin d'augmenter la sensibilité et réaliser la compensation thermique.

Ce transducteur 40 est relié par l'intermédiaire d'une liaison filaire schématiquement illustrée en 41 à un circuit électrique 50 qui délivre à sa sortie 51 un signal électrique dont l'amplitude est proportionnelle à la déformation de flexion verticale de la barre 10, et représente par conséquent les mouvements dits significatifs, comme définis précédemment. Un circuit électronique d'amplification et d'adaptation à la transmission des signaux peut être utilement introduit dans le circuit 50 avant la sortie 51.

De préférence, comme cela est illustré schématiquement sur la figure 1, une butée 60 est prévue en dessous de la barre 10 et à distance de la position occupée par celle-ci au repos. Cette butée 60 qui forme limiteur de course évite les déformations d'amplitude trop grande nuisibles pour la barre 10.

De façon avantageuse, cette butée 60 est formée d'un organe fileté afin d'autoriser un réglage aisé du débattement vertical de la barre 10.

Comme cela est également représenté sur la figure 1, le dispositif précité peut être équipé d'un élément amortisseur 70 supporté par la console 20 et porté au contact de la barre 10 pour amortir les oscillations mécaniques de celle-ci et de sa charge.

Selon le mode de réalisation représenté sur la figure 1, l'élément amortisseur 70 est disposé au-dessus de la barre 10 et supporté par une traverse 23 généralement horizontale et parallèle à la barre 10 et solidaire de l'extrémité supérieure du montant 21 précité.

Bien entendu, le dispositif représenté sur la figure 1 qui comprend un organe de mesure 10-40 sensible aux efforts verticaux transmis par la queue de l'animal est susceptible d'être remplacé par tout autre moyen fonctionnellement équivalent comprenant un organe de suspension et un capteur.

L'un de ces moyens est représenté sur la figure 2 qui va maintenant être décrite.

On notera cependant que le second mode de réalisation du dispositif de saisie d'informations conforme à la présente invention représenté sur la figure 2 ne diffère fondamentalement du premier mode de réalisation représenté sur la figure 1 et précédemment décrit que par la structure de l'organe de suspension 10.

Dans ces conditions, seule la structure de cet organe va maintenant être décrite, les éléments du second mode de réalisation représentés sur la figure 2 qui sont similaires à des éléments précédemment décrits en regard de la figure 1 portent sur les dessins des références numériques incrémentées de 100 en regard de ces éléments homologues.

Selon ce second mode de réalisation, l'organe de saisie d'informations auquel est suspendu l'animal A comprend un levier ou barre rigide 110 (formant organe de suspension) articulé à une première extrémité 111 sur la console 120 autour d'un axe 113, généralement horizontal et transversal à la direction d'élongation de la barre 110.

A son autre extrémité 112, la barre rigide 110 est munie de moyens 130 de fixation par suspension.

L'articulation de la barre 110 sur la console 120 est susceptible là encore de différentes variantes de réalisation.

Selon une première de ces variantes, l'articulation est matérialisée par un tourillon d'axe horizontal engagé d'une part dans un élément de la console 120, d'autre part dans l'extrémité 111 de la barre 110.

Selon une autre variante de réalisation susceptible d'être retenue, l'articulation de la barre 110 sur la console 120 est obtenue par une zone d'affaiblissement (rétrécissement) ménagée dans la barre 110 au voisinage de la console 120.

La barre rigide 110 est de plus associée à un élément élastique 180 sollicitant la barre vers une position neutre de repos. Le cas échéant, l'élément élastique 180 peut être formé d'un ressort spirale accroché d'une part sur la barre 110 au voisinage de la seconde extrémité 112 de celle-ci et d'autre part sur la traverse 123 homologue de la traverse 23 précitée et solidaire du montant 121 de la console.

Bien entendu, le ressort spirale précité 180 est susceptible d'être remplacé par tout moyen fonctionnellement équivalent.

En outre, la barre 110 est là encore associée à un capteur 140 sensible aux efforts verticaux transmis par l'animal à la barre 110. Plus précisément, le capteur 140 est sensible aux déplacements de la barre 110 dans la direction verticale.

Le capteur 140 est relié au circuit électrique 150 grâce à une liaison filaire 141.

On reconnaît par ailleurs sur la figure 2 une butée 160 et un élément amortisseur 170 homologues de la butée 60 et de l'amortisseur 70 décrits en regard de la figure 1.

Là encore, le circuit 150 délivre sur sa sortie 151 éventuellement après une amplification électronique, un signal représentatif des mouvements dits significatifs selon l'invention.

On va maintenant décrire la variante de réalisation illustrée sur les figures 3 à 5.

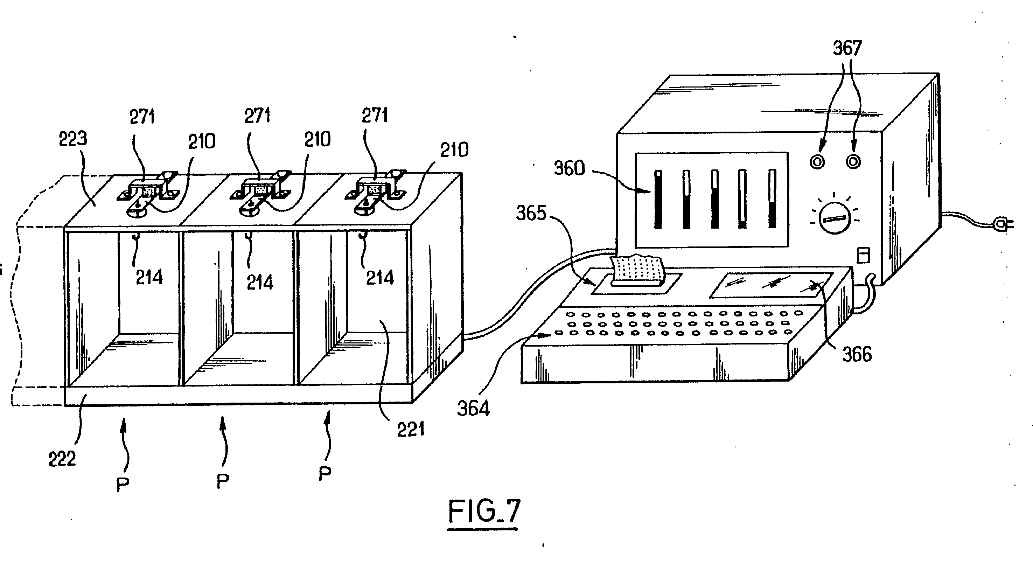

Cette variante de réalisation est adaptée en particulier pour permettre une juxtaposition de plusieurs postes de test , comme illustré sur là gauche de la figure 7, en vue d'une expérimentation simultanée sur plusieurs animaux.

Les éléments du dispositif représentés sur les figures 3 à 5 et 7 portent des références numériques incrémentées de 200 par rapport aux éléments similaires représentés sur la figure 1.

On notera que selon la variante de réalisation représentée sur les figures 3 à 5, la console 220 est constituée d'un boîtier présentant un contour d'ouverture vertical pour l'accrochage et le retrait de l'animal. L'utilisation de tels boîtiers 220 permet d'isoler les animaux adjacents afin d'éviter une perturbation réciproque de ceux-ci.

Plus précisément, le boîtier 220 est formé selon le mode de réalisation représenté sur les figures d'une paroi arrière verticale 221, de deux parois latérales verticales et parallèles 224, 225, d'une paroi inférieure horizontale 222 et enfin d'une paroi supérieure horizontale 223. Bien entendu un couvercle (non représenté sur les figures pour simplifier l'illustration) protègne la partie supérieure du dispositif, en particulier les barres 210.

Une barre flexible 210 à débattement vertical, formant organe de suspension, est fixée au-dessus de la console 220, en position générale horizontale , grâce à une vis 226.

Afin d'autoriser un débattement vertical de la barre 210, cette dernière est disposée à distance de la paroi horizontale supérieure 223 du boîtier 220 grâce à une entretoise 227 engagée sur le corps de la vis 226 entre la plaque 210 et la paroi horizontale supérieure 223 du boîtier.

Cette barre 210 est associée à au moins une jauge de contraintes 240 sensible aux déformations de flexion verticale de la barre et reliée par une liaison filaire 241 au circuit électrique 250.

Au voisinage de son extrémité libre 212, la barre 210 supporte un crochet 214 qui traverse un orifice 228 d'axe vertical ménagé dans la paroi horizontale supérieure 223 et est équipé à son extrémité inférieure d'une pince 215 ou tout autre moyen fonctionnellement équivalent apte à supporter l'animal A, notamment une bande adhésive.

On aperçoit par ailleurs sur la figure 3, une vis 260 engagée dans un alésage taraudé d'axe vertical ménagé dans la paroi horizontale supérieure 223 et faisant saillie au-dessus de celle-ci pour limiter le débattement vertical de la barre 210.

Enfin, on notera la présence d'un élément amortisseur 270 supporté au-dessus de la barre 210 par un étrier 271 fixé sur la plaque horizontale supérieure 223 par des vis 272. Là encore, l'élément 270 a pour but d'amortir les oscillations mécaniques de la barre 210 et de sa charge.

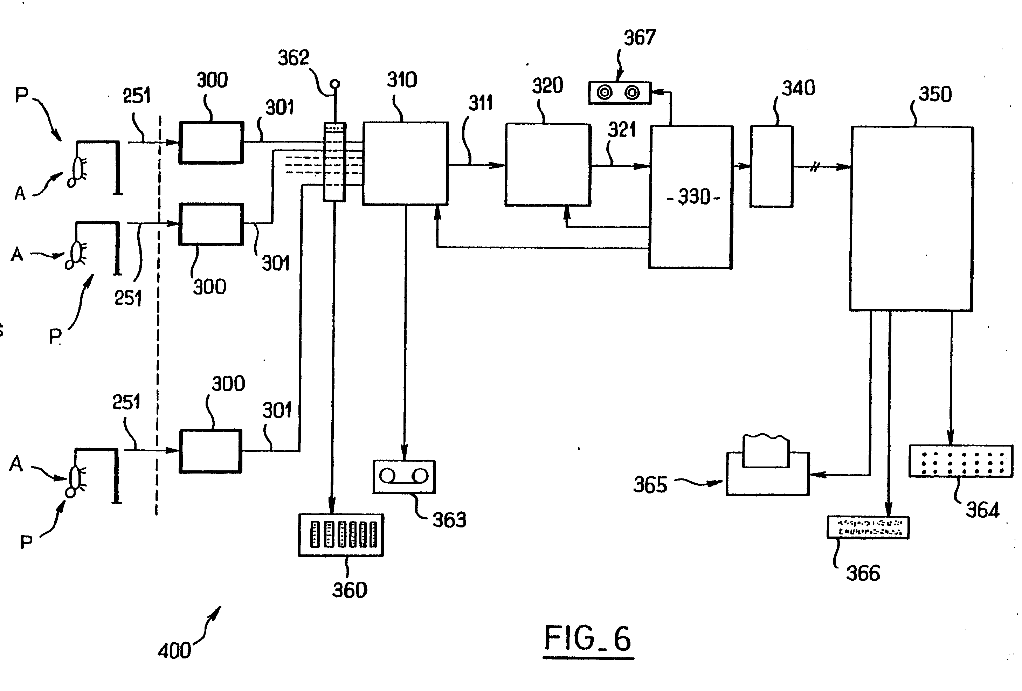

On va maintenant décrire la structure du dispositif électronique 400 représenté sur la figure 6 adapté pour traiter les informations délivrées par une pluralité de postes P de saisie d'informations schématiquement illustrés sur la gauche de cette figure.

Bien entendu, chacun de ces postes P de test ou saisie d'informations comprend un capteur délivrant directement ou après amplification par un circuit électronique un signal représentatif des mouvements dits significatifs de l'animal A.

Les signaux électriques de mesure analogiques qui en résultent, disponibles sur les sorties 251 des circuits électriques 250 sont appliqués respectivement à des éléments 300 d'amplification, de filtrage et de redressement. Les sorties 301 de ces éléments 300 sont multiplexées par un échantillonneur 310 de type cyclique, dont la sortie 311 attaque un convertisseur numérique 320.

La sortie 321 de ce convertisseur est reliée à une unité 330 de traitement informatique. Cette dernière comporte notamment un microprocesseur intégré et tout le logiciel adapté pour assurer en permanence le traitement et le cumul des informations délivrées par les capteurs en vue du calcul de la valeur des paramètres représentatifs de l'agitation significative des animaux pendant le temps d'expérimentation, pour en déduire les effets psychotropes des substances médicamenteuses testées.

Les principales fonctions de calcul de l'unité 330 sont : la mesure et le cumul des durées des phases de mouvements significatifs et/ou d'inactivité, la mesure et le cumul des durées des phases de mouvements significatifs dont l'amplitude dépasse un seuil prédéterminé, le calcul de l'énergie et de la puissance dépensée par l'animal à partir du cumul des carrés des valeurs maximales des amplitudes des mouvements, et la mesure du temps de l'expérimentation.

Le cas échéant, comme cela est représenté sur la figure 6, les informations calculées dans l'unité de traitement 330 peuvent être transmises, en vue d'une exploitation, à un micro-ordinateur 350 par l'intermédiaire d'une unité de transmission par exemple en liaison série 340.

Dans un tel cas, les fonctions principales accomplies par le micro-ordinateur sont : l'entrée des paramètres de calcul et des conditions d'expérimentation, la transmission des ordres de calcul à l'unité de traitement 330, le pilotage des paramètres temporels de l'ensemble fonctionnel et de l'expérimentation, la gestion de différents organes de visualisation et d'impression.

Sur ce point, on notera la possibilité d'utiliser un organe de visualisation 360 comportant une pluralité de postes d'affichage, par exemple des barrettes lumineuses à diodes électroluminescentes ou cristaux liquides connectées respectivement aux sorties 301 des éléments 300 en vue de la visualisation des mouvements significatifs des animaux des divers postes de saisie d'informations.

La connexion ainsi établie au niveau des voies analogiques 301 peut également être utilisée pour sélectionner et connecter l'une de ces voies 301 à une sortie auxiliaire 362 en vue d'une exploitation externe du signal disponible sur cette voie analogique.

Le micro-ordinateur 350 peut de plus contrôler un dispositif d'enregistrement magnétique analogique ou digital 363 relié à l'organe de multiplexage 310 en vue d'un éventuel traitement ultérieur des signaux.

Le micro-ordinateur 350 est de préférence relié à un clavier 364 pour l'entrée des paramètres, des ordres d'expérimentation et de traitement.

Par ailleurs, de préférence, le micro-ordinateur 350 est relié à un dispositif 365 d'impression alphanumérique et graphique pour la sortie des résultats.

De préférence, le système comprend également un dispositif de visualisation alpha-numérique et graphique 366 facilitant le dialogue entre l'opérateur et le micro-ordinateur 350.

Enfin, de façon avantageuse, un dispositif de signalisation par diodes électroluminescentes 367 est raccordé à l'unité de traitement 330 en vue de la visualisation du début et de la fin de la mesure et/ou du traitement pour une période d'expérimentation.

La figure 7 illustre schématiquement un mode de présentation de l'ensemble du dispositif conforme à l'invention, comportant une unité de trois postes de saisie de l'information sur l'animal, les moyens de traitement précités et de visualisation des mouvements significatiis, ainsi que le micro-ordinateur avec son clavier, imprimante et visualisation alpha-numérique.

Bien entendu, le nombre de postes de saisie n'est aucunement limitatif.

Par ailleurs, les moyens de traitement 400 peuvent faire l'objet de nombreuses variantes de réalisation.

De préférence, le microordinateur réalise en option les fonctions suivantes qui visent l'amélioration et la facilité d'expérimentation et d'exploitation des résultats, en particulier lors de tests sur des lots importants d'animaux.

Tout d'abord, lors de l'utilisation d'une pluralité de postes de saisie, le microordinateur détermine et visualise pour l'opérateur qui suspend les animaux sur les organes de suspension, une répartition aléatoire des animaux sur lesdits postes de saisie, en vue de diminuer les erreurs introduites par les dispositifs de mesure.

De plus, en vue de l'analyse statistique des résultats obtenus sur un ensemble d'expérimentations réalisées sur un lot important d'animaux, traités avec différentes doses de substances médicamenteuses, le microordinateur met en mémoire et classifie les résultats des mesures individuelles. A la fin de l'ensemble de l'expérimentation, le microordinateur effectue automatiquement les calculs statistiques et comparatifs des résultats de mesure pour chaque paramètre spécifique du procédé décrit précédemment et pour chaque dose de médicament. Le microordinateur enregistre pour chaque médicament, pour chaque paramètre et pour chaque dose de médicament, les tableaux récapitulatifs des résultats individuels (de chaque animal) des mesures, ainsi que les résultats statistiques et leur représentation graphique.

Les résultats statistiques sont constitués, par exemple, et à titre non limitatif par : la moyenne des résultats individuels, l'écart type ou l'écart à la moyenne, le test de DUNNETT.

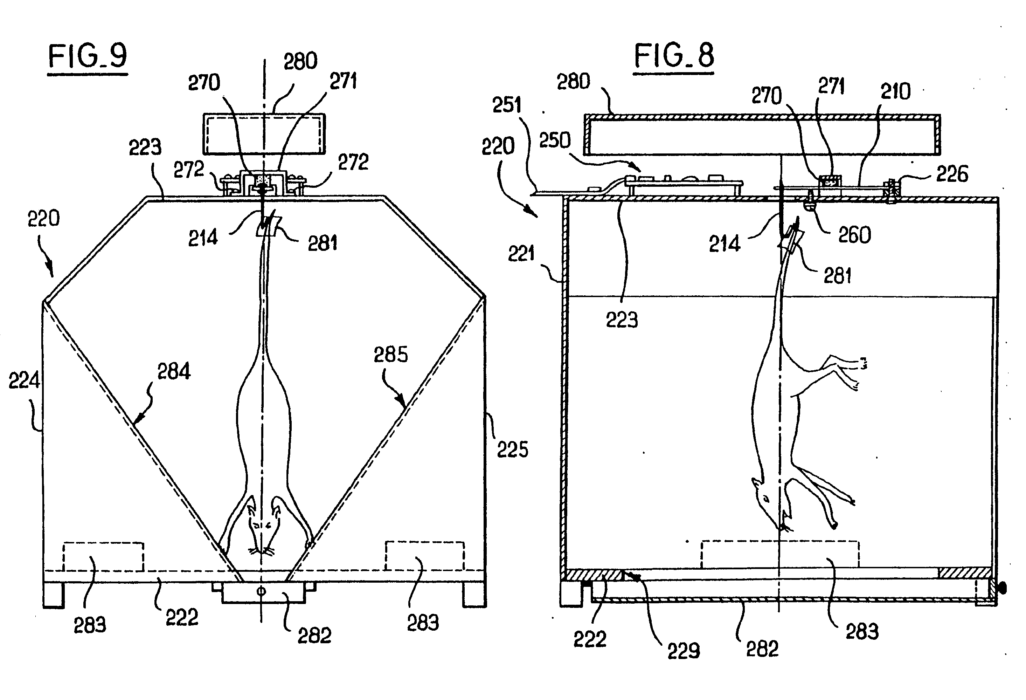

La présente invention propose par ailleurs une variante de réalisation du dispositif de saisie d'informations illustrée sur les figures 8 et 9, qui est adaptée spécialement pour l'expérimentation sur des animaux relativement lourds, notamment sur les rats.

Cette variante de réalisation reprend la majeure partie des éléments illustrés sur les figures 3 et 4 et précédemment décrits.

Cette variante de réalisation ne sera donc pas décrite en détail par la suite et comportera sur les figures 8 et 9 des références numériques identiques à celles utilisées sur les figures 3 et 4.

La différence essentielle distinguant la variante représentée sur les figures 8 et 9 du mode de de réalisation illustré sur les figures 3 et 4 réside dans l'utilisation d'un appui pour l'animal visant à alléger l'effort dû au poids de l'animal appliqué sur le membre suspendu de celui-ci.

Le cas échéant, la surface d'appui peut être généralement horizontale.

Néanmoins, de préférence, la surface d'appui est formée d'un ou plusieurs plans inclinés 284, 285 sur lesquels l'animal suspendu par la queue ou par tout autre membre peut s'appuyer par ses membres antérieurs sans pouvoir s'y accrocher.

Dans le cas de l'utilisation de deux plans inclinés 284, 285 comme illustré sur la figure 9, ces plans sont de préférence symétriques par rapport à un plan vertical parallèle à la direction d'élongation de la barre 210, les plans inclinés divergeant vers le haut et présentant entre eux une ouverture angulaire comprise entre 30 et 75°. Bien entendu, la surface d'appui des plans inclinés sur laquelle repose l'animal est soigneusement polie.

On distingue par ailleurs sur les figures 8 et 9 une ouverture 229 réalisée dans la paroi inférieure 222 et un tiroir sous-jacent amovible 282 pour l'évacuation des excréments produits par l'animal pendant l'expérimentation.

On distingue également le couvercle 280 protégeant les éléments sensibles du dispositif, tels que la barre 210 et le circuit 250, une bande adhésive 281 permettant de suspendre la queue de l'animal sur le crochet 214 et des poids 283 reposant contre la paroi inférieure 222 pour élever la stabilité du dispositif.

Bien entendu, de préférence, de façon similaire à la réprésentation de la figure 7 plusieurs unités du dispositif de saisie représenté sur les figures 8 et 9 sont assemblées pour constituer un ensemble présentant plusieurs postes d'expérimentation simultanée.

## Revendications

1. Procédé d'évaluation des effets psychotropes de substances médicamenteuses chez les animaux, comprenant les étapes consistant, après avoir administré les substances médicamenteuses à un animal:

i) à suspendre celui-ci de préférence par la queue, sur un organe de suspension (10, 110, 210) équipé d'un capteur (40, 140, 240) sensible aux efforts verticaux transmis par l'animal à cet organe, lors de son agitation,

ii) à détecter, pendant un temps d'expérimantation prédéterminé, le signal généré par le capteur (40, 140, 240), signal qui constitue la mesure des mouvements de l'animal liés à un déplacement du centre de gravité de celui-ci dans la direction verticale et représente par conséquent les mouvements significatifs de l'animal car impliquant une dépense importante d'énergie, et

iii) à déterminer, par traitement du signal généré par le capteur (40, 140, 240), la valeur d'un paramètre représentatif de l'agitation significative de l'animal(A) pendant le temps d'expérimentation.

2. Procédé d'évaluation des effets psychotropes de substances médicamenteuses selon la revendication 1, caractérisé par le fait que le capteur (40, 140, 240) est sensible à la flexion ou aux déplacements de l'organe de suspension dans la direction verticale.

3. Procédé d'évaluation des effets psychotropes de substances médicamenteuses selon l'une des revendications 1 ou 2, caractérisé par le fait que l'étape iii) de détermination de la valeur du paramètre représentatif de l'agitation significative de l'animal (A) pendant le temps d'expérimentation consiste :

– à mesurer la durée des phases élémentaires d'agitation significative de l'animal (A) détectées par le capteur (40, 140, 240), et

– à réaliser la somme des durées des phases élémentaires d'agitation significative détectées pendant le temps d'expérimentation, cette somme étant représentative de la valeur recherchée du paramètre.

4. Procédé d'évaluation des effets psychotropes de substances médicamenteuses selon l'une des revendications 1 ou 2, caractérisé par le fait que l'étape iii) de détermination de la valeur du paramètre représentatif de l'agitation significative de l'animal pendant le temps d'expérimentation consiste :

a) à mesurer les durées de phases d'inactivité de l'animal, en considérant comme phase d'inactivité tout état de l'animal en dehors des phases de mouvement significatif, et

b) à réaliser la somme des durées des phases d'inactivité détectées pendant le temps d'expérimentation, cette somme étant considérée représentative de la valeur recherchée du paramètre.

5. Procédé d'évaluation des effets psychotropes de substances médicamenteuses selon l'une des revendications 1 à 4, caractérisée par le fait que l'étape iii) de détermination de la valeur du paramètre représentatif de l'agitation significative de l'animal (A) pendant le temps d'expérimentation consiste :

a) à comparer le signal généré par le capteur (40, 140, 240) à une valeur de seuil pour retenir les signaux représentatifs d'un mouvement significatif dont les amplitudes dépassent un seuil prédéterminé,

b) à mesurer les durées des phases de mouvements significatifs ou des phases d'inactivité à partir des signaux retenus à l'étape a) et

c) à réaliser la somme des durées des phases de mouvements significatifs ou des phases d'inactivité détectées à l'étape b) pendant le temps d'expérimentation, ces sommes étant représentatives des valeurs recherchées du paramètre.

6. Procédé d'évaluation des effets psychotropes de substances médicamenteuses selon l'une des revendications 1 et 2, caractérisé par le fait que l'étape iii) de détermination de la valeur du paramètre représentatif de l'agitation significative de l'animal (A) pendant le temps d'expérimentation consiste :

    a) à mesurer les amplitudes des signaux générés par le capteur (40, 140, 240), correspondant aux mouvements élémentaires significatifs de l'animal (A),

    b) à élever les valeurs des amplitudes de ces signaux au carré.

    c) à opérer la somme des carrés obtenus à l'étape b) pour évaluer l'énergie totale dépensée pendant le temps d'expérimentation, cette énergie totale étant représentative de la valeur recherchée du paramètre.

7. Procédé d'évaluation des effets psychotropes de substances médicamenteuses selon l'une des revendications 1 à 4, caractérisé par le fait que l'étape iii) de détermination de la valeur du paramètre représentatif de l'agitation significative de l'animal pendant le temps d'expérimentation consiste :

    a) à mesurer les valeurs des amplitudes des signaux électriques générés par le capteur, correspondant aux efforts verticaux exercés sur l'organe de suspension par les mouvements significatifs de l'animal,

    b) à élever au carré les valeurs mesurées des amplitudes, ce carré étant considéré ici comme représentatif de l'énergie dépensée par l'animal pendant chaque mouvement significatif élémentaire,

    c) à effectuer la somme des carrés obtenus à l'étape b) afin d'évaluer l'énergie totale dépensée par l'animal pendant le temps d'expérimentation, et

    d) à diviser la somme des carrés obtenus à l'étape c) par le temps total d'agitation significative de l'animal, la valeur de ce quotient étant considérée comme représentative de la valeur recherchée du paramètre.

8. Dispositif d'évaluation des effets psychotropes de substances médicamenteuses chez les animaux pour la mise en oeuvre du procédé selon l'une des revendications 1 à 7, qui comprend :

    – au moins un dispositif de saisie d'informations comportant :

        . un organe de suspension (10, 110, 210) muni de moyens aptes à permettre la suspension d'un animal, de préférence par la queue, et

        . un capteur (40, 140, 240) sensible aux efforts verticaux transmis par l'animal à cet organe de suspension, lors de son agitation, et liés à un déplacement du centre de gravité de l'animal dans la direction verticale,

    – des moyens de traitement (400) du signal généré par le capteur pendant un temps d'expérimentation prédéterminé aptes à évaluer l'agitation significative de l'animal pendant ce temps, et

    – des moyens de visualisation (360, 365) de la valeur d'un paramètre représentatif de cette agitation significative.

9. Dispositif selon la revendication 8, caractérisé par le fait qu'il comprend :

    – une pluralité de dispositifs de saisie d'information comportant chacun un organe de suspension (10, 110, 210) et un capteur (40, 140, 240),

    – un organe de multiplexage (310) recevant les signaux délivrés par les capteurs qui attaque

    – des moyens de traitement (320, 330, 350) des signaux générés par ces capteurs pendant un temps d'expérimentation prédéterminé, aptes à évaluer l'agitation significative de chaque animal pendant ce temps, et

    – des moyens de visualisation (360, 365) des valeurs déterminées par les moyens de traitement.

10. Dispositif selon l'une des revendications 8 ou 9, caractérisé par le fait que certains au moins des dispositifs de saisie d'informations comprennent un élément support (284, 285) servant d'appui à l'animal suspendu pour alléger l'effort dû au poids de l'animal exercé sur le membre suspendu.

11. Dispositif selon la revendication 10, caractérisé par le fait que l'élément support comprend au moins un plan incliné (284, 285) sur lequel de préférence l'animal suspendu prend appui par ses membres antérieurs sans pouvoir s'accrocher.

12. Dispositif selon l'une des revendications 8 à 11, caractérisé par le fait que l'organe de suspension comprend une barre flexible (10, 210) généralement horizontale fixée sur une embase (21, 221) par une première extrémité (11, 211) et munie de moyens de suspension d'animal (30 ; 214, 215) au voisinage de sa seconde extrémité (12, 212), le capteur (40, 240) étant formé d'un transducteur sensible à la déformation de flexion verticale de la barre.

13. Dispositif selon la revendication 12, caractérisé par le fait que le transducteur (40, 240) comprend au moins une jauge de contraintes.

14. Dispositif selon l'une des revendications 8 à 11, caractérisé par le fait que l'organe de suspension comprend une barre rigide (110) généralement horizontale articulée sur une embase (121) autour d'un axe horizontal (113) transversal à sa direction longitudinale, par une première extrémité (111), et munie de moyens de suspension d'animal (130) au voisinage de sa seconde extrémité, la barre (110) étant associée à un élément élastique (180) sollicitant la barre vers une position neutre de repos.

15. Dispositif selon l'une des revendications 8 à 14, caractérisé par le fait qu'il comprend en outre un amortisseur (70, 170, 270) limitant les oscillations mécaniques de l'organe de suspension (10, 110, 210).

**Patentansprüche**

1. Verfahren zur Bewertung der psychotropen Effekte von heilkräftigen Substanzen bei Tieren, das nach der Verabreichung der heilkräftigen Substanzen an ein Tier die folgenden Stufen umfaßt :

(i) das Aufhängen des Tieres, vorzugsweise am Schwanz, an einer Aufhängeeinrichtungen (10,110,210), die mit einem Sensor (40,140,240) ausgestattet ist, der empfindlich für vertikale Belastungen ist, die von dem Tier bei seiner Bewegung auf diese Einrichtung ausgeübt werden,

(ii) die Messung des von dem Sensor (40,140,240) erzeugten Signals während eines vorgegebenen Versuchszeitraums, wobei das Signal das Maß für die Bewegungen des Tieres ist, die mit einer Verschiebung des Gravitationszentrums desselben in vertikaler Richtung verbunden sind und infolgedessen die signifikanten Bewegungen des Tieres repräsentiert, die einen bedeutenden Energieverbrauch mit sich bringen, und

(iii) die Festlegung des Wertes eines für die signifikante Bewegung des Tieres (A) während des Versuchszeitraums repräsentativen Parameters durch Behandlung des von dem Sensor (40,140,240) abgegebenen Signals.

2. Verfahren zur Bewertung der psychotropen Effekte von heilkräftigen Substanzen nach Anspruch 1, dadurch gekennzeichnet, daß der Sensor (40,140,240) empfindlich ist für eine Verbiegung oder für Verschiebungen der Aufhängeeinrichtung in vertikaler Richtung.

3. Verfahren zur Bewertung der psychotropen Effekte von heilkräftigen Substanzen nach einem der Ansprüchen 1 und 2, dadurch gekennzeichnet, daß die Stufe (iii) zur Festlegung des Wertes des Parameters, der für die signifikante Bewegung des Tieres (A) während des Versuchszeitraums repräsentativ ist, darin besteht, daß man :

– die Dauern der elementaren Phasen der signifikante Bewegung des Tieres (A), die von dem Sensor (40,140,240) gemessen werden, bestimmt und

– die Summe der Dauern der elementaren Phasen der signifikanten Bewegung, die während des Versuchszeitraums bestimmt worden sind, bildet, wobei diese Summe repräsentativ für den gesuchten Wert des Parameters ist.

4. Verfahren zur Bewertung der psychotropen Effekte von heilkräftigen Substanzen nach einem der Ansprüche 1 und 2, dadurch gekennzeichnet, daß die Stufe (iii) zur Festlegung des Wertes des Parameters, der für die signifikante Bewegung des Tieres während des Versuchszeitraums repräsentativ ist, darin besteht, daß man :

(a) die Dauernder Inaktivitätsphasen des Tieres mißt, wobei man als Inaktivitätsphase jeden Zustand des Tieres außerhalb der signifikanten Bewegungsphasen ansieht, und

(b) die Summe der Dauernder während des Versuchszeitraums gemessenen Inaktivitätsphasen bildet, wobei diese Summe als repräsentativ für den gesuchten Wert des Parameters angesehen wird.

5. Verfahren zur Bewertung der psychotropen Effekte von heilkräftigen Substanzen nach einem der Ansprüche 1 bis 4, dadurch gekennzeichnet, daß die Stufe (iii) zur Festlegung des Wertes des Parameters, der für die signifikante Bewegung des Tieres (A) während des Versuchszeitraums repräsentativ ist, darin besteht, daß man :

(a) das von dem Sensor (40,140,240) erzeugte Signal mit einem Schwellenwert vergleicht, um die für eine signifikante Bewegung repräsentativen Signale festzuhalten, deren Amplituden einen vorgegebenen Schwellenwert übersteigen,

(b) die Dauer der signifikanten Bewegungsphasen oder der Inaktivitätsphasen, ausgehend von den in der Stufe (a) festgehaltenen Signalen, mißt und

(c) die Summe der Dauern der in der Stufe (b) während des Versuchszeitraums gemessenen signifikanten Bewegungsphasen oder Inaktivitätsphasen bildet, wobei diese Summen repräsentativ sind für die gesuchten Werte des Parameters.

6. Verfahren zur Bewertung der psychotropen Effekte von heilkräftigen Substanzen nach einem der Ansprüche 1 und 2, dadurch gekennzeichnet, daß die Stufe (iii) zur Festlegung des Wertes des Parameters, der für die signifikante Bewegung des Tieres (A) während des Versuchszeitraums repräsentativ ist, darin besteht, daß man

(a) die Amplituden der von dem Sensor (40,140,240) entsprechend den signifikanten elementaren Bewegungen des Tieres (A) erzeugten Signale mißt,

(b) die Werte der Amplituden dieser Signale ins Quadrat erhebt und

(c) die Summe der in der Stufe (b) erhaltenen Quadrate verwendet zur Bewertung der während des Versuchszeitraums verbrauchten Gesamtenergie, wobei diese Gesamtenergie repräsentativ ist für den gesuchten Wert des Parameters.

7. Verfahren zur Bewertung der psychotropen Effekte von heilkräftigen Substanzen nach einem der Ansprüche 1 bis 4, dadurch gekennzeichnet, daß die Stufe (iii) zur Festlegung des Wertes des Parameters, der für die signifikante Bewegung des Tieres während des Versuchszeitraums repräsentativ ist, darin besteht, daß man

(a) die Werte der Amplituden der von dem Sensor elektrisch erzeugten Signale, die den vertikalen Belastungen entsprechen, die durch die signifikanten Bewegungen des. Tieres auf die Aufhängeeinrichtung ausgeübt werden, mißt,

(b) die gemessenen Werte der Amplituden ins Quadrat erhebt, wobei dieses Quadrat hier als repräsentativ für die von dem Tier während jeder signifikanten elementaren Bewegung verbrauchte Energie angesehen wird,

(c) die Summe der in der Stufe (b) erhaltenen Quadrate bildet zur Bewertung der von dem Tier während des Versuchszeitraums verbrauchten Gesamtenergie und

(d) die Summe der Quadrate, die in der Stufe (c) erhalten wird, durch die Gesamtzeit der signifikanten Bewegung des Tieres dividiert, wobei der Wert dieses Quotienten als repräsentativ für den gesuchten Wert des Parameters angesehen wird.

8. Vorrichtung zur Bewertung der psychotropen Effekte von heilkräftigen Substanzen bei Tieren zur Durchführung des Verfahrens nach einem der Ansprüche 1 bis 7, die umfaßt :

   – mindestens eine Einrichtung zum Erfassen von Informationen, die umfaßt :

     . eine Aufhängeeinrichtung (10,110,210), die mit Einrichtungen ausgestattet ist, die das Aufhängen eines Tieres, vorzugsweise am Schwanz, erlauben, und

     . einen Sensor (40,140,240), der empfindlich ist für vertikale Belastungen, die von dem Tier auf diese Aufhängeeinrichtung übertragen werden bei seiner Bewegung und die mit einer Verschiebung des Gravitationszentrums des Tieres in der vertikalen Richtung verbunden sind,

   – Einrichtungen (400) zur Behandlung des von dem Sensor erzeugten Signals während eines vorgegebenen Versuchszeitraums, die geeignet sind, die signifikante Bewegung des Tieres während dieses Zeitraums zu bewerten, und

   – Einrichtungen (360,365) zum Sichtbarmachen des Wertes eines Parameters, der für diese signifikante Bewegung repräsentativ ist.

9. Vorrichtung nach Anspruch 8, dadurch gekennzeichnet, daß sie umfaßt :

   – eine Vielzahl von Einrichtungen zur Erfassung von Informationen, die jeweils eine Aufhängeeinrichtung (10,110,210) und einen Sensor (40,140,240) umfassen,

   – eine Multiplexier-Einrichtung (310), welche die von den Sensoren abgegebenen Signale aufnimmt, die antreibt

   – Einrichtungen (320,330,350) zum Behandeln der von diesen Sensoren während eines vorgegebenen Versuchszeitraums erzeugten Signalen, die geeignet sind, die signifikante Bewegung jedes Tieres während dieses Zeitraums zu bewerten, und

   – Einrichtungen (360,365) zum Sichtbarmachen der durch die Behandlungseinrichtungen bestimmten Werte.

10. Vorrichtung nach einem der Ansprüche 8 und 9, dadurch gekennzeichnet, daß bestimmte mindestens der Einrichtungen zur Erfassung der Informationen ein Trägerelement (284,285) umfassen, das als Auflage für ein aufgehängtes Tier dient, um die Belastung als Folge des Gewichts des Tieres, die auf die Aufhängeeinrichtung ausgeübt wird, zu verringern.

11. Vorrichtung nach Anspruch 10, dadurch gekennzeichnet, daß das Trägerelement mindestens eine geneigte Ebene (284, 285) aufweist, auf der sich vorzugsweise das aufgehängte Tier durch seine vorderen Gliedmaßen abstützt, ohne sich daran festhalten zu können.

12. Vorrichtung nach einem der Ansprüche 8 bis 11, dadurch gekennzeichnet, daß die Aufhängeeinrichtung umfaßt eine flexible Stange (10,210), die mit einem ersten Ende (11,211) im allgemeinen horizontal auf einer Befestigungsfläche (21,221) befestigt ist und mit Einrichtungen zum Aufhängen des Tieres (30 ; 214, 215) in der Nähe des zweiten Endes (12,212) ausgestattet ist, wobei der Sensor (40,240) durch einen Signalumformer gebildet wird, der für die vertikale Biegeverformung der Stange empfindlich ist.

13. Vorrichtung nach Anspruch 12, dadurch gekennzeichnet, daß der Signalumformer (40,240) mindestens ein Dehnungsmeßgerät umfaßt.

14. Vorrichtung nach einem der Ansprüche 8 bis 11, dadurch gekennzeichnet, daß die Aufhängeeinrichtung umfaßt eine starre Stange (110), die durch ein erstes Ende (111) im allgemeinen horizontal angelenkt ist an einer Befestigungsfläche (121), so daß sie sich um eine horizontale Achse (113) transversal zu ihrer Längsrichtung frei bewegen kann, und die mit Einrichtungen zum Aufhängen des Tieres (130) in der Nähe ihres zweiten Endes versehen ist, wobei die Stange (110) mit einem elastischen Element (180) verbunden ist, das die Stange in eine neutrale Ruheposition zwingt.

15. Vorrichtung nach einem der Ansprüche 8 bis 14, dadurch gekennzeichnet, daß sie außerdem eine Dämpfungseinrichtung (70,170,270) umfaßt, welche die mechanischen Schwingungen der Aufhängeeinrichtung (10,110,210) begrenzt.

## Claims

1. A method for evaluating the psychotropic effects of medicinal substances on animals, comprising the stages of, after having administered medicinal substances to an animal :

    i) suspending the animal, preferably by the tail, on suspension means (10, 110, 210) fitted with a detector (40, 140, 240) which is sensitive to the vertical forces transmitted by the animal to such means when it is agitated,

ii) detecting the signal generated by the sensor (40, 140, 240) over a predetermined experimental period, the signal constituting a measurement of the movements of the animal associated with displacement of the latter's centre of gravity in the vertical direction and thus representing significant movement by the animal, implying a major expenditure of energy, and

iii) determining the value of a parameter representing significant agitation of the animal (A) during the experimental period through processing of the signal generated by the detector (40, 140, 240).

2. A method for evaluating the psychotropic effects of medicinal substances in accordance with claim 1, characterised in that the sensor (40, 140, 240) is sensitive to bending of the suspension means or its displacement in the vertical direction.

3. A method for evaluating the psychotropic effects of medicinal substances in accordance with either of claims 1 or 2, characterised in that stage iii) of determining the value of the parameter representing significant agitation of the animal (A) during the experimental period consists of :

– measuring the duration of unit phases of significant agitation of the animal (A) detected by the sensor (40, 140, 240), and

– summing the durations of the unit phases of significant agitation detected during the experimental period, this sum being representative of the required value of the parameter.

4. A method for evaluating the psychotropic effects of medicinal substances in accordance with either of claims 1 or 2, characterised in that stage iii) of determining a value of the parameter representing significant agitation of the animal during the experimental period consists of :

a) measuring the duration of phases during which the animal is inactive, regarding an inactive phase as any state of the animal when not in a phase of significant movement, and

b) summing the durations of the phases of inactivity detected during the experimental period, this sum being regarded as being representative of the required value of the parameter.

5. A method for evaluating the psychotropic effects of medicinal substances in accordance with one of claims 1 to 4, characterised in that stage iii) of determining the value of the parameter representing significant agitation of the animal (A) during the experimental period consists of :

a) comparing the signal generated by the detector (40, 140, 240) with a threshold value in order to identify signals representing significant movement of an amplitude exceeding a predetermined threshold,

b) measuring the durations of the phases of significant movement or phases of inactivity using the signals recognised in stage a) and

c) summing the durations of the phases of significant movement or phases of inactivity detected in stage b) during the experimental period, these sums being representative of the required values of the parameter.

6. A method for evaluating the psychotropic effects of medicinal substances in accordance with either of claims 1 or 2, characterised in that stage iii) of determining the value of the parameter representing significant agitation of the animal (A) during the experimental period consists of :

a) measuring the amplitudes of the signals generated by the detector (40, 140, 240) corresponding to significant unit movements by the animal (A),

b) squaring the values of the amplitudes of the signals,

c) summing the squares obtained in stage b) to evaluate the total energy expended during the experimental period, this total energy being representative of the required value of the parameter.

7. A method for evaluating the psychotropic effects of medicinal substances in accordance with one of claims 1 to 4, characterised in that stage iii) of determining the value of the parameter representing significant agitation of the animal during the experimental period consists of :

a) measuring the values of the amplitudes of the electrical signals generated by the detector corresponding to vertical forces exerted on the suspension means by significant movements by the animal,

b) squaring the measured values of the amplitudes, this square here being regarded as being representative of the energy expended by the animal during each significant unit movement,

c) summing the squares obtained in stage b) in order to evaluate the total energy expended by the animal during the experimental period, and

d) dividing the sum of the squares obtained in stage c) by the total time for significant agitation of the animal, the value of this quotient being regarded as being representative of the required value of the parameter.

8. Means for evaluating the psychotropic effects of medicinal substances in animals using the method described in any one of claims 1 to 7, comprising :

– at least one information input device comprising :

. suspension means (10, 110, 210) fitted with means capable of suspending an animal, preferably by the tail, and

. a detector (40, 140, 240) which is sensitive to vertical forces transmitted by the animal to this suspension means when agitated, and associated with displacement of the centre of gravity of the animal in the vertical direction,

– means for processing (400) the signal generated by the detector during a predetermined experimental period suitable for evaluating significant agitation of the animal during this period, and

– means for displaying (360, 365) the value of a parameter representing this significant agitation.

9. Means according to claim 8, characterised in that it comprises :

– a plurality of information input devices each incorporating suspension mans (10, 110, 210) and a detector (40, 140, 240),

– multiplexing means (310) receiving signals produced by the sensors activated,

– means for processing (320, 330, 350) the signals generated by these detectors during a predetermined experimental period capable of evaluating significant agitation of each animal during this period, and

– means for displaying (360, 365) the values determined by the processing means.

10. Means according to either of claims 8 or 9, characterised in that at least some of the information input devices comprise a supporting member (284, 285) providing a support for the suspended animal in order to reduce the force due to the weight of the animal exerted on the suspended member.

11. Means according to claim 10, characterised in that the supporting member comprises at least one inclined plane (284, 285) on which the animal which is suspended can preferably gain support from its front limbs without being able to hold on.

12. Means according to one of claims 8 to 11, characterised in that the suspension member comprises a flexible bar (10, 210) which is generally horizontal and is fixed on a base (21, 221) at one end (11, 211) and is equipped with means for suspending the animal (30 ; 214, 215) in the vicinity of its other end (12, 212), the sensor (40, 240) consisting of a transducer which is sensitive to vertical bending deformation of the bar.

13. Means according to claim 12, characterised in that the transducer (40, 240) includes at least one strain gauge.

14. Means according to one of claims 8 to 11, characterised in that the suspension member comprises a rigid bar (110) which is generally horizontal and is articulated on a base (121) about a horizontal axis (113) transverse to its longitudinal direction at one end (111) and is provided with means for suspending the animal (130) in the vicinity of its other end, the bar (110) being associated with an elastic member (180) which stresses the bar towards a neutral resting position.

15. Means according to one of claims 8 to 14, characterised in that it also comprises a damper (70, 170, 270) restricting mechanical oscillations of the suspension means (10, 110, 210).

FIG_1

FIG_2

FIG_3

FIG_4

FIG_5

FIG. 6

EP 0 214 169 B1

FIG.7

EP 0 214 169 B1

FIG_9

FIG_8

EP 0 214 169 B1